# EUROPEAN PATENT APPLICATION

(11) **EP 1 429 275 A2**
(43) Date of publication of application: **16.06.2004**
(21) Application number: 03257588.8
(22) Date of filing: 02.12.2003
(51) Int. Cl.: G06F 19/00

(54) **Improvements in or relating to data processing systems**

(30) Priority: 11.12.2002 GB 0228960
(71) Applicant: Mirada Solutions Ltd, Oxford OX1 2ET (GB)
(72) Inventor: Behrenbruch, Christian Peter, Oxford OX1 2ET (GB); Declerck, Jerome Marie Joseph, Oxford OX1 2ET (GB); Brady, John Michael, Oxford OX1 2ET (GB)
(74) Representative: Nicholls, Michael John

(57) **Abstract**

A processing system which comprises a processing apparatus and a processing agent which is administered to a processing subject. The processing agent has a primary behaviour which provides the desired process result in conjunction with the apparatus, but also has a distinctive signature characteristic which is detected by test functionality in the processing system. The behaviour of the processing system can be modified in response to the test result. In an example such as a contrast enhanced medical imaging, the full functionality of the imaging equipment may be available only if a contrast agent having the particular distinctive signature characteristic is used, thus tying use of the apparatus to use of a particular agent.

## Description

The present invention is concerned with processing systems, in particular systems in which a processing apparatus is used in conjunction with a processing agent administered to a processing subject to achieve a desired process result.

There are many situations in which to achieve a desired process result on a processing subject it is necessary to use a combination of both a processing apparatus and also a processing agent which is administered to the processing subject. Examples of this abound in the field of analysis where a subject to be analysed first has some analysis agent administered to it, and during or after the administration, an analysis apparatus is used to reveal some condition of the analysis subject. This may be *in vivo,* where a typical example might be medical imaging where a contrast agent is administered to the subject or patient, and then an imaging apparatus used to image the patient or subject, or *in vitro*, for example in an assay where some agent is added to the analight before it is analysed by an analysis apparatus.

Many processing systems are now software controlled. Software may be provided in the processing apparatus, or in some data processor separate from the processing apparatus, which is effective to improve the processing so as to achieve an improved process result. For example in the imaging example mentioned above, an imaging apparatus may be provided with image processing software which processes the imaging results and provides them in an improved form to the clinician or user. Often a considerable amount of time and effort is invested in preparing such software and so the traditional methods of restricting use of the software to licenced users are used, such as:
- passwords/software keys/licence number authentication
- hardware locks (hardware keys "dongles")
- processor/network/hardware identification.
Incidently, in this specification the use of the word "software" is intended to cover not only software, but firmware and other process controlled, automation and analysis systems, including those in which the functionality is achieved by the use of dedicated circuitry, rather than general purpose data processors running computer programs.

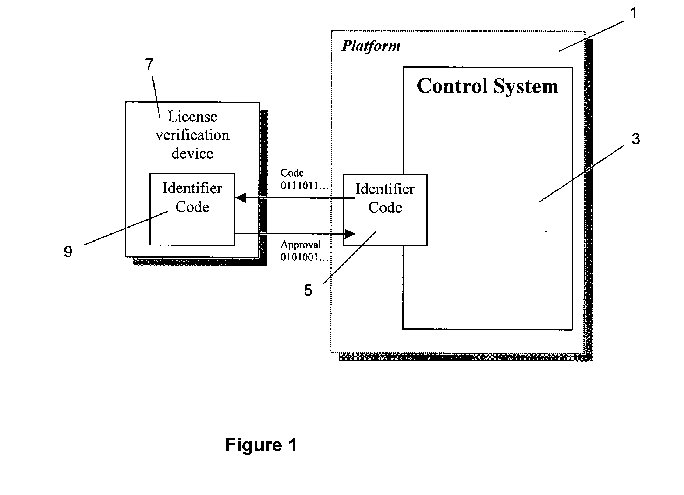

Figure 1 illustrates schematically the conventional approach to securing the distribution of software systems such as licence identification, serial numbers, hardware locks ("dongles") etc. Thus in Figure 1 a processing apparatus 1 includes a control system 3 which is provided with authentication functionality 5. This authentication functionality 5 communicates with a licence verification device 7, which includes an identifier code 9, and on provision of the correct identification to the licence verification device 7, approval is given back to the processing apparatus 1 for it to function. This process may be similar to the familiar one of registering software with the software manufacturer in order for it to be enabled, or may be based on the use of a "dongle" which is a small hardware device which is attached to an information interface of an apparatus, which restricts the use of the apparatus unless the dongle is attached.

The present invention is concerned with providing an alternative way of protecting or authorising use of a processing system where that processing system is designed to be used with a processing agent. In particular the invention provides for the processing agent to have a particular distinctive signature which distinguishes it from other processing agents, and for the system to include test functionality to interrogate the processing agent to ensure that its signature behaviour is consistent with that expected. The signature behaviour may be inherent to the processing agent, or added to it, or provided in another component which is administered along with the processing agent.

Thus in more detail, the present invention provides a processing system comprising an processing apparatus and a processing agent, the processing agent being administrable to a processing subject and having in relation thereto a primary behaviour effective in combination with said processing apparatus to achieve a desired process result, wherein the processing agent further has a distinctive signature characteristic distinguishing it from other processing agents, and wherein the processing system comprises test functionality to test for the distinctive signature characteristic of the processing agent and selectively to modify subsequent operation of the processing apparatus based on the test result.

The test functionality may be effective to disable, at least partially, subsequent operation of the processing apparatus in the absence of the distinctive signature characteristic. It may be effective, at least partially, to disable output of the process result.

The processing agent may comprise a first component for providing the primary behaviour and a second component having the distinctive signature characteristic. The two components may be mixed, or the two may be bound at a molecular level. The signature characteristic may be in the behaviour of the processing agent in the processing subject, such as its time-dependent or spatial behaviour, or it may be a property of the processing agent which is detectable by the processing apparatus.

In the case of the processing system being an analysis system, the processing apparatus is an analysis apparatus, the processing agent is an analysis agent and the processing subject is an analysis subject. The primary behaviour of the analysis agent is effective to reveal on analysis by the analysis apparatus a condition of the analysis subject. In this case the distinctive signature characteristic may be a property of the analysis agent detectable in the course of the analysis of the primary behaviour of the analysis agent. Thus additional analysis operations are unnecessary. In one example, such as an imaging apparatus, one image acquisition is effective both to acquire the image and to acquire data allowing the distinctive signature characteristic to be tested for. In this example the analysis agent may be a contrast agent.

The analysis agent may, for example, comprise two radioisotopes of different decay characteristics, which provide the distinctive signature characteristic, or may comprise a component emitting photons of a particular energy to provide the distinctive signature characteristic, or a component having a distinctive magnetic resonance spectrum.

The processing subject may be a human being, plant or animal, or the processing may be *in vitro.*

The invention extends to a computer program which comprises program code means for providing the test functionality, and optionally also for controlling the apparatus. Further, the invention extends to a processing agent for use in such a processing system and which is provided with a distinctive signature characteristic in addition to its primary behaviour, thus allowing it to be distinguished from other processing agents by the test functionality.

The invention will be further described by way of example with reference to the accompanying drawings in which:-
Figure 1 schematically illustrates a traditional authentication process;
Figure 2 schematically illustrates an authentication process according to one embodiment of the present invention;
Figure 3 schematically illustrates an authentication process according to another embodiment of the present invention;
Figure 4 illustrates a particular example of a distinctive signature characteristic in a processing agent;
Figure 5 illustrates another example of a distinctive signature characteristic in a processing agent; and
Figure 6 illustrates the magnetic resonance spectra for different molecules.

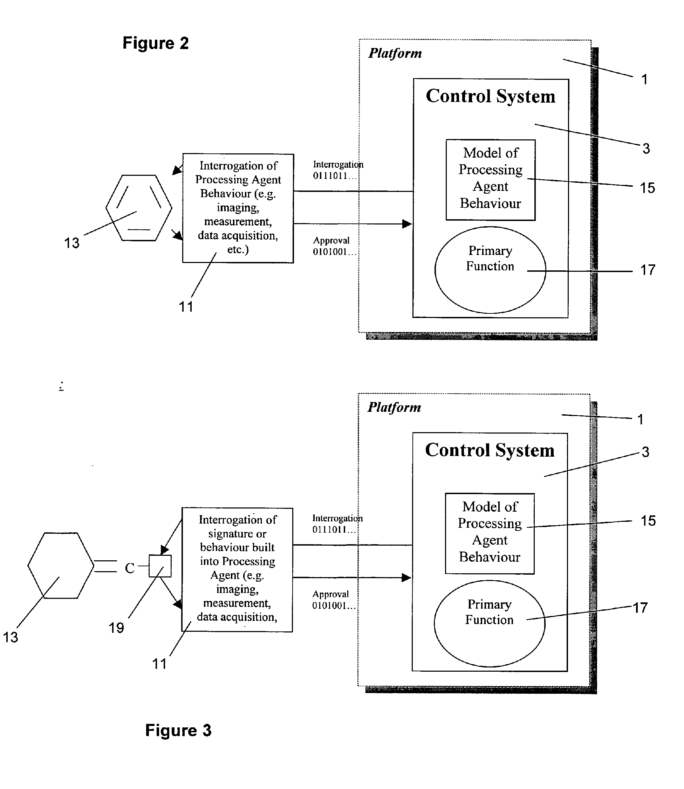

Figure 2 schematically illustrates a first embodiment of the invention. A processing apparatus 1 includes a control system 3 which is responsible for controlling the processing apparatus to achieve a desired processing result in conjunction with a processing agent 13, which may be a product for administration to a processing subject. The system includes test functionality illustrated here as 11 and 15 for interrogating the behaviour of the processing agent and comparing the result to a model 15 of the behaviour of the processing agent. If the result is positive then the primary function 17 of the apparatus is enabled.

Figure 3 illustrates an alternative embodiment in which the processing agent 13 has bound to it another component 19 which provides the distinctive signature behaviour. Otherwise the operation of the embodiment of Figure 3 is the same as that of Figure 2.

In the event that it is not possible to modify the processing agent in the manner illustrated in Figure 3, the additional component providing the distinctive signature behaviour may simply be mixed with the processing agent. As an example, in the field of magnetic resonance imaging contrast agents such as gadolinium chelates are used for contrast-enhancement. These may be mixed with a benign synthetic protein having a specific nuclear magnetic resonance signal (spectrum) which can be detected by the magnetic resonance scanner as part of the acquisition process. The scanner may be designed to enable output of the results only if the required signal of the benign synthetic protein is present. Alternatively, it may be that basic results are output with any contrast agent, regardless of the test result, but that additional processing to enhance the image is only available if the contrast agent including the benign synthetic protein is used.

Various ways of providing a distinctive signature characteristic in the processing agent will be explained below in relation to the field of medical imaging by SPECT, PET and MRI.

**Single Photon Emission Computed Tomography** cameras detect disintegration of photons from nuclear isotope sources. Many radioactive isotopes are used in practice, for instance ^{99m}Tc (Technetium), or ²⁰¹T1 (Thallium). The disintegration of the isotope creates photons of a particular energy, which depends of the isotope and the energy level of the reaction. For instance, for ^{99m}Tc the energy of the emitted photon is between 135 and 145 keV, for ²⁰¹T1, the energy is between 60 and 85 keV. There are a couple of dozen different radio-isotopes used in clinical practice in nuclear medicine departments, and all generate photons of different energies (say between 50 and 200 keV), but the gamma cameras of the SPECT machines can detect all these photons and can detect the energy of the photon which has reached the detector.

Therefore to provide a distinctive signature characteristic a contrast agent is prepared (similar to that for a dual isotope acquisition) which contains two different isotopes A and B, for example A may be ²⁰¹T1 and B may be ^{99m}Tc. Isotope A is the one used for the clinical diagnostic protocol, i.e. providing the primary behaviour of the analysis agent, and B provides the distinctive signature characteristic. The presence and amount of isotope B is kept secret to the manufacturer of the contrast agent and the software system controlling the SPECT apparatus. Providing for the SPECT apparatus to detect the proportion of isotope B present means that the apparatus can be enabled only in the presence of the expected proportion of isotope B, or the full functionality can be provided only in the presence of the expected proportion of agent B. The detection of the radiation from the two isotopes may be achieved during the same acquisition protocol, without the need for any additional operations by the user.

**Positron Emission Tomography** detectors can identify the photon pair emitted from the disintegration of a positron, itself emitted from a radioactive isotope such as ¹⁸F or¹⁵O. The energy of the positron is 511 keV, and although there is no possibility to differentiate a positron emitted from ¹⁸F or from ¹⁵O, the half-life of the two isotopes are different: for ¹⁸F, around 2 hours, for ¹⁵O, around 2 minutes.

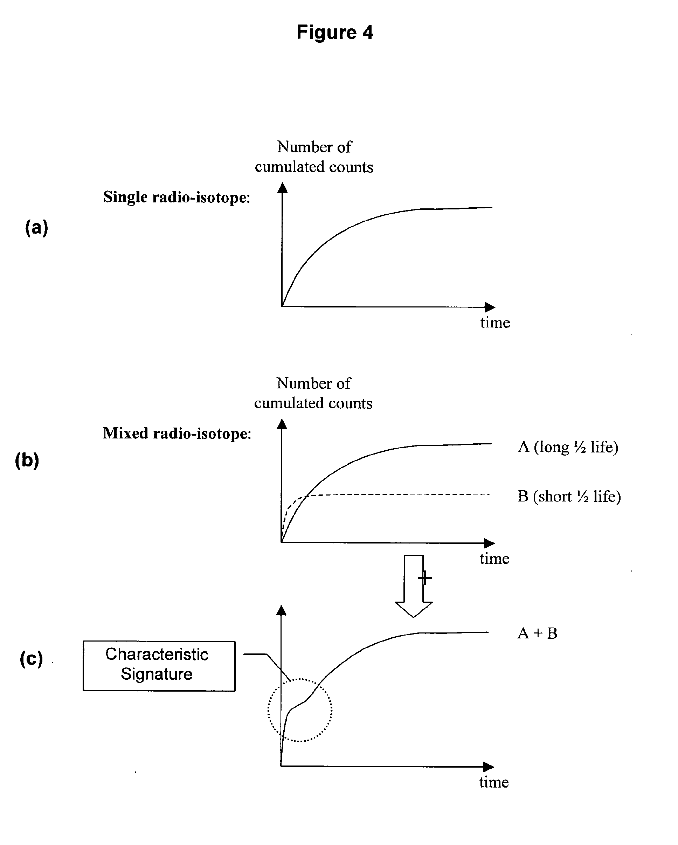

Thus a contrast agent can be prepared as a combination of known proportions of the two different agents ¹⁸F and ¹⁵O; the diagnostic agent is ¹⁸F associated for instance with the deoxy-glucose (FDG), the other one, ¹⁵O, with water. When the patient is placed in the PET machine, photons are counted. For a single contrast agent injection, the number of photons emitted (and therefore, detected), follow an exponential decay, whose characteristic time is the half-life of the isotope. If two radio-isotopes are injected, the number of count emitted per second is just the sum of these two exponentials. In the PET detector the cumulative number of counts is detected. Figure 4 (a) shows the cumulative count for a single radio-isotope. In Figure 4 (b) the cumulative counts for the two different agents having the two different half-lives are shown separately. The sum, which is what the detector detects is shown in Figure 4 (c). It will be seen that the result of including the two different radioisotopes is that there is a characteristic kink in the shape of the curve. This constitutes the distinctive signature characteristic for this contrast agent. Thus the PET camera and image processing software can test for this kink (e.g. by differentiating the curve and detecting the gradient change) and, on finding it, the proper functionality is provided to the user.

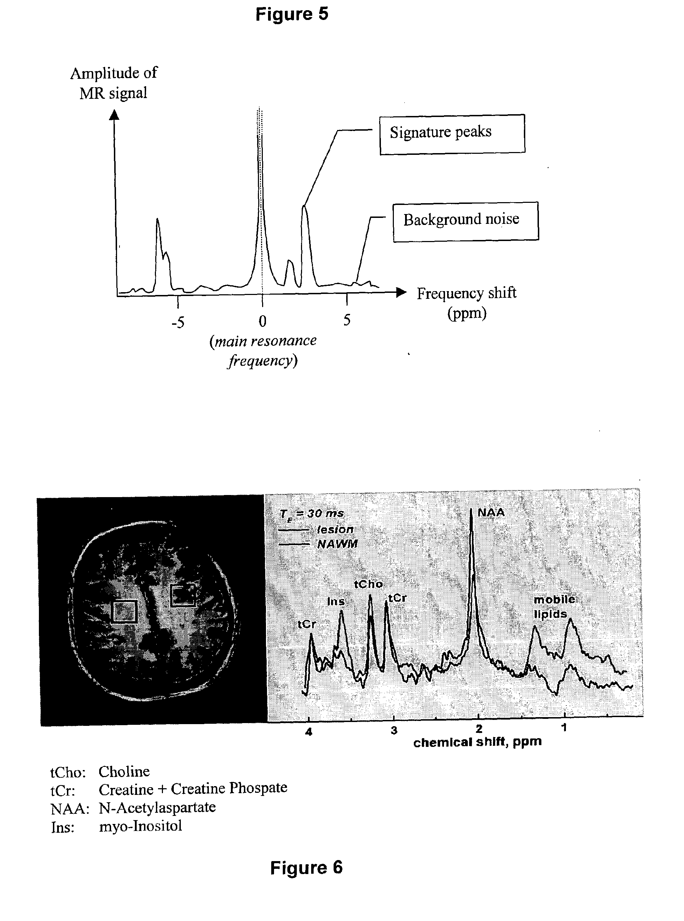

In the field of **magnetic resonance imaging and magnetic resonance spectroscopy**, the distinctive magnetic resonance spectrum of the contrast agent can form the distinctive signature characteristic. Figure 5 illustrates a magnetic resonance spectrum for a particular molecule. As indicated in Figure 5, the position and height of peaks above the background noise form a distinctive signature for the molecule. Magnetic resonance imaging and spectroscopy commonly use contrast agents and such contrast agents can be combined with (either by simple mixing or molecular binding) another molecule which has a clear and distinctive magnetic resonance spectrum. For example, Figure 6 illustrates the spectra for various different molecules and the presence of the different molecules can be recognised from the different curves. (Figure 6 in fact shows two MRS curves, one in a lesion (marked "lesion") and one in a normal area of white matter (marked "NAWM"). The peaks identified and labelled indicate unequivocally the presence of the four types of molecule listed below the graph, namely: tCho - Choline; tCr - Creatine + Creatine Phosphate; NAA - N-acetylaspartate; and Ins - myo-Inositol). As with the examples above, the presence of a particular molecule included in the processing agent is used as a "key" to unlock the desired functionality of the processing apparatus.

Although the examples above have been described with reference to medical imaging, the invention is applicable to other analysis techniques and indeed broadly to processing systems which use a processing agent administered to a processing subject together with a processing apparatus. By using the distinctive signature characteristic of the processing agent to unlock the functionality of the processing apparatus, this enables the use and functionality of particular apparatus to be tied with use of particular processing agents. It can therefore ensure that only approved processing protocols are used, giving quality control, and control of the use of third party apparatus and agents.

## Claims

1. A processing system comprising an processing apparatus and a processing agent, the processing agent being administrable to a processing subject and having in relation thereto a primary behaviour effective in combination with said processing apparatus to achieve a desired process result, wherein the processing agent further has a distinctive signature characteristic distinguishing it from other processing agents, and wherein the processing system comprises test functionality to test for the distinctive signature characteristic of the processing agent and selectively to modify subsequent operation of the processing apparatus based on the test result.

2. A processing system according to claim1 wherein the test functionality is effective to disable, at least partially, subsequent operation of the processing apparatus in the absence of said distinctive signature characteristic.

3. A processing system according to claim 1 or 2 wherein the test functionality is effective to disable, at least partially, output of the process result in the absence of said distinctive signature characteristic.

4. A processing system according to claim 1, 2 or 3 wherein the processing agent comprises a first component for providing said primary behaviour and a second component having said distinctive signature characteristic.

5. A processing system according to any one of the preceding claims wherein the distinctive signature characteristic is in the behaviour of the processing agent in the processing subject.

6. A processing system according to claim 5 wherein the distinctive signature characteristic is in the time-dependent behaviour of the processing agent in the processing subject.

7. A processing system according to claim 5 or 6 wherein the distinctive signature characteristic is in the spatially-dependent behaviour of the processing agent in the processing subject.

8. A processing system according to any one of the preceding claims wherein the distinctive signature characteristic is a property of the processing agent detectable by the processing apparatus.

9. A processing system according to any one of the preceding claims wherein the processing apparatus comprises an analysis apparatus, the processing agent comprises an analysis agent and the processing subject is an analysis subject, the analysis agent being administrable to the analysis subject and having in relation thereto a primary behaviour effective to reveal upon analysis by the analysis apparatus a condition of the analysis subject as said process result.

10. An analysis system according to claim 9 wherein the distinctive signature characteristic is a property of the processing agent detectable by the analysis apparatus on analysis of the primary behaviour of the analysis agent.

11. An analysis system according to claim 9 or 10 wherein the analysis apparatus is a medical imaging apparatus and the analysis agent comprises a contrast agent.

12. An analysis system according to claim 11 wherein the analysis agent comprises two radio isotopes of different decay characteristics to provide said distinctive signature characteristic.

13. An analysis system according to claim 11 wherein the analysis agent comprises a component emitting photons of a particular energy to provide said distinctive signature characteristic.

14. An analysis system according to claim 11 wherein the distinctive signature characteristic is the magnetic resonance spectrum of the contrast agent.

15. An analysis system according to claim 11 wherein the analysis agent comprises the contrast agent and a further component having a distinctive magnetic resonance spectrum to provide said distinctive signature characteristic.

16. A processing system according to any one of the preceding claims wherein the processing subject is a human being, plant or animal.

17. A processing system according to any one of the preceding claims wherein the processing subject is *in vitro*.

18. A computer program comprising program code means for providing on a programmed data processor test functionality for use in a processing system in accordance with any one of claims 1 to 17.

19. A computer program according to claim 18 further comprising program code means for controlling said processing apparatus to achieve said desired process result.

20. A processing agent for use in a processing system in accordance with any one of claims 1 to 17 and having in relation to a predetermined processing subject a primary behaviour effective in combination with said processing apparatus to achieve a desired process result, and further having a distinctive signature characteristic distinguishing it from other processing agents and distinguishable by said test functionality.
